# EUROPÄISCHE PATENTSCHRIFT

(11) **EP 3 073 913 B1**
(45) Veröffentlichungstag und Bekanntmachung des Hinweises auf die Patenterteilung: **27.02.2019**
(21) Anmeldenummer: 14828128.0
(22) Anmeldetag: 14.11.2014
(51) Int. Cl.: A61B 5/055, A61B 6/04

(54) **PATIENTENTISCH ZUR HERSTELLUNG VON NMR- UND/ODER CT-AUFNAHMEN**
PATIENT TABLE FOR ESTABLISHING NMR RECORDINGS AND/OR CT RECORDINGS
LIT D'EXAMEN POUR LA RÉALISATION DE PRISES DE VUE PAR RMN ET/OU TOMODENSITOMÉTRIE

(30) Priorität: 29.11.2013 DE 102013113273
(43) Veröffentlichungstag der Anmeldung: 05.10.2016
(73) Patentinhaber: Noras, Hubert, 97082 Würzburg (DE)
(72) Erfinder: Noras, Hubert, 97082 Würzburg (DE)
(74) Vertreter: Pöhner, Wilfried Anton
(86) Internationale Anmeldenummer: PCT/DE2014/100404
(87) Internationale Veröffentlichungsnummer: WO 2015/078447

(56) Entgegenhaltungen:
- WO-A1-2004/071302
- WO-A1-2014/079415
- US-A1- 2009 308 400

## Beschreibung

Die Erfindung bezieht sich auf einen Patiententisch für eine optimale Ausrichtung der abzubildenden Körperpartien eines Probanden in einer NMR-Röhre oder CT-Röhre, wobei der Patiententisch in axialer Richtung in die Röhre einfahrbar ist wobei eine Brücke (2) vorhanden ist, die Außenabmessungen der Brücke (2) in radialer Richtung kleiner gewählt sind als der lichte Durchmesser der Röhre, ein Gurt vorhanden ist, der mit seinem einen Ende an der Brücke (2) befestigt und in axialer Richtung spann- und fixierbar ist.

In der modernen medizinischen Diagnostik sind Röntgen- oder NMR-Aufnahmen zu einem sehr wichtigen Diagnosemittel geworden. Die Röntgentomographie ist ein bildgebendes Verfahren zur Darstellung einer Schicht eines zu untersuchenden Objekts, wobei die modernen Verfahren mehrere Schichten aufnehmen und mithilfe eines Computers auswerten, die aus diesem Grund als computertomographische Verfahren bezeichnet werden. Ohne die Möglichkeiten der unterschiedlichen Aufnahmetechniken und deren jeweilige Vor- und Nachteile im Einzelnen abzuhandeln, besteht das Grundprinzip darin, in ein als Hohlzylinder ausgebildetes und gemeinhin als Röhre bezeichnendes Gerät den zu untersuchenden Proband auf einem Patiententisch liegend in Richtung der Achse der Röhre einzufahren und dort in der Weise zu positionieren, dass sich die abzubildende Körperpartie etwa in der Mittelebene der Röhre befindet. Das abzubildende Objekt wird dann aus vielen Richtungen von Röntgenstrahlen durchleuchtet und das Absorptionsprofil des jeweils bestrahlten Objekts gemessen. Im Anschluss daran erfolgt dann die Computergestützte Bildrekonstruktion, die Abbildungen der entsprechenden Transversalschnitte des Untersuchungsobjektes errechnet.

Auf völlig anderen physikalischen Prinzipien beruht die Magnetresonanztomographie (MRT) oder auch häufig Kernspintomographie genannt. Auch hierdurch werden Schnittbilder des zu untersuchenden Objekts erzeugt, die eine diagnostische Beurteilung der abgebildeten Körperpartie zulässt. Das Grundprinzip besteht darin, dass Atomkerne, bei denen es sich meist um Wasserstoffkerne handelt, in einem starken Magnetfeld mit magnetischen Wechselfeldern angeregt werden, um dann bei Rückkehr in den Grundzustand ein Energiequant zu emittieren, dessen Frequenz durch die punktuell herrschende Magnetfeldstärke bestimmt ist. Der Bildkontrast entsteht dadurch, dass verschiedene Gewebe einen unterschiedlichen Gehalt an Wasserstoffatomen aufweisen und dementsprechend unterschiedlich Intensität an Energiequanten emittieren. Auch bei diesem Diagnoseverfahren wird der Proband auf einem Patiententisch liegend in eine hohlzylindrisch gestaltete Röhre eingefahren.

Den vorgeschilderten Untersuchungsverfahren ist gemeinsam, dass nur jene Körperpartien, in denen anatomische Anomalien vermutet werden, aus ökonomischen Gesichtspunkten einer Erfassung unterzogen werden. Dabei wird der Proband in der Horizontalen ausgestreckt auf dem Patiententisch liegend in die Röhre eingefahren. Bei bestimmten Fragestellungen ist es von Vorteil, wenn die Lage des Probanden von der ausgestreckten Horizontalen abweicht. Hierzu zählen Aufnahmen der Wirbelsäule und des Kopfes, das Anwinkeln der Beine bei Untersuchungen urologischer und gynäkologischer Art, was dadurch geschieht, dass Kissen oder Stützen anderer Art unter den Probanden oder im Falle des Anwinkelns der Beine unter die Oberschenkel verbracht werden. Nachdem diese Unterstützungsmaßnahmen außerhalb der Röhre an auf dem Patiententisch liegenden Probanden vorgenommen werden, ergibt sich das räumliche Problem, dass die Veränderung der Position und z. B. das Anheben der Unterschenkel nur um eine solche Strecke erfolgen darf, die sicherstellt, dass der Patient problemlos in die Röhre eingeschoben werden kann. Es muss vermieden werden, dass Knie oder Oberschenkel oder Kopf soweit in radialer Richtung überstehen, dass eine Kollision mit der Innenkante der Röhre erfolgt. Es ist deshalb bekannt, vor dem Einfahren in die Röhre mithilfe einer Schablone auszumessen und festzustellen, ob das kollisionsfreie Einfahren in die Röhre möglich wird.

Die Veröffentlichungsschrift WO 2004/071302 A1 beschreibt eine Armunterstützung zur Positionierung der Arme eines liegenden Patienten während eines CT Scans in Form einer bogenförmig den Thorax überspannenden Brücke mit oberseitigen Depressionen zur Aufnahme der Arme. Die Arme können durch Bänder überspannt und so fixiert werden.

Diese Positionierungshilfe ist zum einen nur für die Arme geeignet und zum anderen kann die Höhe oder Relative Stellung der Arme auch nicht variiert werden.

Die Offenlegungsschrift US 2009/0308400 A1 beschreibt ein System zur Positionierung eines Patienten umfassend einen Patiententisch und verschiedene brückenartige Positionierungshilfen.

Diese erlauben jedoch sämtlich nicht, eine zu untersuchenden Körperregion in Höhe und/oder Ausrichtung variabel anzuheben.

Die Erfindung hat sich die Aufgabe gestellt, eine Vorrichtung zu schaffen, die zum einen einzelne Körperpartien unter- und abzustützen erlaubt und zum anderen jedoch ein kollisionsfreies Einfahren in die Röhre sicherstellt.

Gelöst wird diese Aufgabe dadurch, dass eine Brücke vorhanden ist, die sich auf den Längsrändern des Patiententisches in einander gegenüberliegenden Punkten abstützt, die Außenabmessungen der Brücke in radialer Richtung kleiner gewählt sind als der lichte Durchmesser der Röhre, ein Gurt vorhanden ist, der mit seinem einen Ende an der Brücke befestigt, unter dem Patienten hindurch geführt und wieder zurück zur Brücke geführt ist und der Gurt in axialer Richtung spann- und fixierbar ist.

Der Kerngedanke der Erfindung besteht darin, eine Vorrichtung zu schaffen, die eine Aufhängung der zu untersuchenden und abzubildenden Körperpartien auf dem Patiententisch außerhalb der Röhre in der gewünschten Position vorzunehmen erlaubt und anschließend der Patiententisch in die Röhre eingefahren werden kann und Kollisionen mit Sicherheit ausgeschlossen sind. Zu diesem Zweck ist eine Brücke vorgesehen, die sich mit ihrem einen Ende auf dem einen Rand des Patiententisches und mit ihrem anderen Ende auf der gegenüberliegenden Seite des Patiententisches randseitig abstützt und in ihrer äußeren Abmessung so gewählt ist, dass sie geringer ist als die lichte Weite der Röhre. Die anzuhebenden Körperpartien werden in der Weise festgelegt, dass sie von Gurten untergriffen werden und die Gurte mit ihren beiden Enden an der Brücke lösbar befestigt sind. Der Gurt selbst kann in axialer Richtung gespannt und fixiert werden, wobei das Spannen und/oder Fixieren manuell oder unter Zuhilfenahme von Spannvorrichtungen erfolgen kann.

Der räumliche Verlauf eines Gurtes ist wie folgt:
Das äußere Ende wird an der Brücke festgelegt. Von dort ausgehend verläuft der Gurt zu dem anzuhebenden Körperteil, umschreibt diesen in etwa halbkreisförmig und wird wiederum nach oben zur Brücke geführt, um dort um einen Umlenkpunkt herumgelegt zu werden. Der Umlenkpunkt ist in etwa entsprechend der Breite des anzuhebenden Körperteils von dem Befestigungspunkt distanziert. Von dort wird der Gurt weiter geführt entlang der Außenseite der Brücke um in der Spannvorrichtung zu enden, die gleichzeitig als Fixiervorrichtung dient. Die Aufgabe der Spannvorrichtung (oder eine entsprechende manuelle Aktivität) ist, durch axiales Verkürzen der Gurte das Anheben des jeweiligen Körperteils zu bewirken und zwar bis in eine solche Höhe, in welche die Körperpartie die zur Aufnahme gewünschte Position annimmt. Die Körperpartie befindet sich somit in einem Raum eingegrenzt, der nach oben hin durch den Verlauf der Brücke und nach unten bzw. den dazwischen befindlichen Seitenflächen hin durch die Gurte begrenzt ist. Somit ist es sichergestellt, dass die jeweilige Körperpartie eine solche räumliche Anordnung erfährt, dass das Einfahren der Patientenliege in die Röhre mit Sicherheit kollisionsfrei erfolgen kann, ohne dass eine Überprüfung der Außenabmessungen mithilfe von Schablonen vorgenommen werden muss. Weiter erlaubt die Vorrichtung das Anheben des entsprechenden Körperteils von der Oberfläche der Patientenliege nach oben zu, wo die Hubbewegung spätestens dann, wenn die nach oben weisende Fläche des Körperteils an der Brücke von unten her zur Anlage kommt endet. Ein weiterer Vorteil ist die universelle Einsetzbarkeit der erfindungsgemäßen Vorrichtung. So kann die Brücke im Bereich des Kopfes angeordnet werden, um den Kopf anzuheben und auszurichten und optimale Aufnahme von bestimmten Röntgenbereichen des Kopfes und/oder auch der Wirbelsäule zu gestatten. Auch wäre es möglich den Oberkörper anzuheben und auszurichten, ebenso können Becken und Beine angehoben werden. Kontaktflächen mit dem Gurt sind dann die Oberschenkel, bei deren Anheben das Bein ein Beugen im Bereich von Knie und Hüfte erfährt und damit sicherstellt, dass sich Ober- und Unterschenkel innerhalb des durch die Brücke begrenzten Raumes befinden. Die Tatsache, dass sich der Fuß über die durch die Brücke definierte Fläche hinaus erstrecken kann, wie es bei weitgehender Ausnutzung des zur Verfügung stehenden Anhebewegs des Unterschenkels der Fall ist, bleibt ohne Bedeutung, da auch im eingefahrenen Zustand der Patientenliege die Füße außerhalb der Röhre zu liegen kommen.

Für die Beantwortung zahlreicher Fragestellungen bzw. die Anfertigung optimaler Aufnahmen auf dem Gebiet der Urologie und Gynäkologie ist das Anheben beider Beine und auf dem Gebiet der Orthopädie das Anheben beider Arme erwünscht. Zwar lassen sich in einem solchen Fall beide Beine bzw. Arme durch einen einzigen Gurt anheben, dessen räumlicher Verlauf so orientiert wäre, dass er sowohl das eine Bein (bzw. den einen Arm) und nach einer Umlenkung im Bereich der Brücke das andere Bein (bzw. den Arm) untergreift. Durch Anspannen der Gurte lassen sich beide Beine gleichzeitig und in Abhängigkeit voneinander einstellen, eine Positionierung der Beine bzw. Arme unabhängig voneinander ist jedoch vielfach gewünscht. Zur Lösung wird vorgeschlagen, dass zwei Gurte in Richtung des Umfangs hintereinander angeordnet sind, die jedoch in ein und derselben Ebene verlaufen, die durch die Brücke beschrieben wird und senkrecht zur Längsachse des Patiententisches ausgerichtet ist. Jeder der einzelnen Gurte steht dann eine eigene Spannvorrichtung zur Verfügung, so dass eine problemlose Einstellung individuell und unabhängig voneinander vorgenommen werden kann.

In einer Weiterbildung wird vorgeschlagen, dass in einer Brücke zwei parallel zueinander verlaufende Gurte angeordnet werden. Die Gurte sind dann in einem und derselben Brücke untergebracht jedoch in Achsrichtung der Brücke bzw. Längsrichtung der Patientenliege gegeneinander versetzt. Zur Verdeutlichung soll dies anhand des Beispiels der Unterstützung eines Unterschenkels bei der Anhebung eines Beines verdeutlicht werden. Die beiden parallelen Gurte bilden zwei Schlaufen, die in Längsrichtung der Patientenliege voneinander beabstandet sind, so dass sich zwei Unterstützungspunkte für denselben Unterschenkel ergeben.

Die Vorteile sind in mehrerer Hinsicht gegeben:
So erlaubt diese Weiterbildung eine Ausrichtung des jeweiligen Unterschenkels in jeder beliebiger Richtung zum Beispiel in der Horizontalen aber auch in einem bestimmten, hiervon abweichenden Anstellwinkel. Zum anderen bewirkt die Abstützung des Unterschenkels in zwei Punkten eine positionssichere Lage für die Dauer der Aufnahme. Bei Unterstützung nur in einem Punkt könnte es zu Bewegungen des Unterschenkels um den Unterstützungspunkt kommen, mit der Folge von Unschärfen der Aufnahme.

Der Anstellwinkel kann auch in der Weise verändert werden, dass der fußnahe Gurt den Oberschenkel weiter nach außen zieht, als der Benachbarte, so dass die Beine eine Spreizung erfahren d. h. in eine V-förmige Stellung übergehen, durch die der Zugang zum Unterkörper erleichtert wird. Zu großem Vorteil erweist sich diese Position dann, wenn chirurgische Werkzeuge zu Zwecken der Operation und/oder Biopsie, die Zugabe von Kontrastmitteln und/oder das Infundieren von Kältemitteln vorgesehen ist, wobei letztere zur Apoptose von Gewebe, wie zum Beispiel von Tumorgewebe, eingesetzt werden.

Aufgrund der Tatsache, dass die vorgeschlagene Vorrichtung bei der Herstellung von Aufnahmen unterschiedlichster Körperpartien eingesetzt und verwendet werden können, aber auch aufgrund der Tatsache, dass die Größe und Abmessungen der Probanden stark variiert, empfiehlt sich, die Brücken auf dem Patiententisch verfahrbar anzuordnen. Eine optimale Ausrichtung der Brücke auch dann, wenn sich der Patient bereits auf der Liege befindet, wird dann möglich.

Grundsätzlich denkbar ist, dass gleichzeitig mehr als eine Brücke auf ein und demselben Patiententisch angebunden sind. Die Möglichkeit der gewünschten Ausrichtung einzelner Körperpartien zur Optimierung der Aufnahme werden durch Verwendung einer zweiten oder mehrerer Brücken beträchtlich erweitert.

Durch die Brücke und die daran befestigten Gurte soll die Qualität der Aufnahmen selbstredend nicht beeinträchtigt werden. Durch Wahl entsprechender Materialien, die für die jeweils zu übermittelnden Signale, bei denen es sich im Falle von Röntgen bzw. Computertomographien um Röntgensignale und bei NMR-Aufnahmen um Radiowellen handelt, transparent sind, wird dieses Ziel erreicht. Hier wird im Speziellen für das NMR die Verwendung des Materials GFK vorgeschlagen, bei dem es sich bekanntlich um Glasfaserverstärkten Kunststoff handelt.

Mitunter wird von den Probanden die Gewichtsaufnahme in den durch den Gurt bestimmten Abstützpunkten aufgrund der vergleichsweise hohen lokalen Kräfte als unangenehm empfunden. Es wird deshalb als zweckmäßig vorgeschlagen, dass sich zwischen dem Gurt und der Körperpartie eine Schale befindet, die dem lokalen Verlauf der Körperpartie formmäßig entsprechend angepasst ist. Dann verteilt sich die Kraftfluss auf eine größere Fläche und führt zu einer Verteilung der Abstützkräfte, was als angenehm für den Proband empfunden wird. Für den speziellen Fall, dass die Unterschenkel angehoben werden sollen, wird eine Schale vorgeschlagen, die von der Form eines Zylindermantels ist. Im Zusammenhang mit vorliegender Erfindung ist hierunter ein Hohlzylinder zu verstehen, der in Richtung seiner Zylinderachse in zwei Halbschalen aufgetrennt ist.

Weitere Einzelheiten, Merkmale und Vorteile der Erfindung lassen sich dem nachfolgenden Beschreibungsteil entnehmen, in dem anhand der Zeichnung ein Ausführungsbeispiel der Erfindung näher erläutert wird. Es zeigen:
- **Figur 1**: einen Patiententisch mit der erfindungsgemäß vorgeschlagenen Brücke
- **Figur 2**: die Brücke in Detaildarstellung.

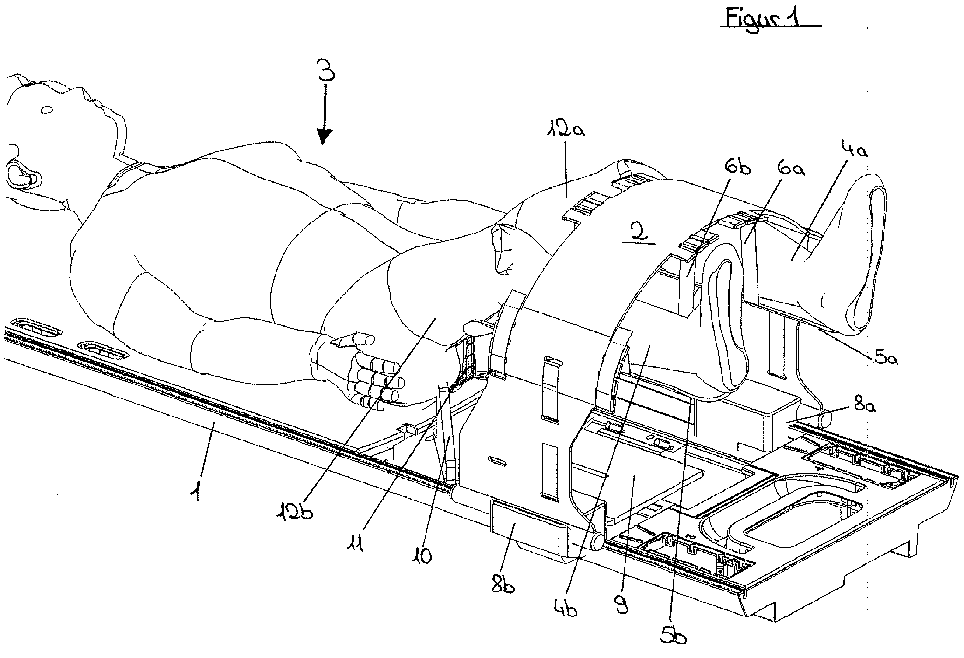

Die **Figur 1** zeigt in perspektivische Darstellung einen Patiententisch (1), auf dem sich die erfindungsgemäße Brücke (2) befindet sowie ein Proband (3) in der Untersuchungsposition, so dass der Patiententisch (1) bereit steht, um in die (nicht dargestellte) Röhre eingefahren zu werden. Die Zeichnung zeigt ein Anwendungsbeispiel, bei dem mit Hilfe der Brücke (2) die Unterschenkel (4a, 4b) über die durch den Patiententisch (1) gebildete Ebene hinaus angehoben werden. Zu diesem Zweck liegen die Unterschenkel auf jeweils einer Beinschale (5a, 5b) auf. Die Beinschalen (5) mit den Unterschenkeln (4) werden über Gurte (6) angehoben bzw. gehalten, die in der Brücke (2) gehalten werden.

Im folgenden wird der räumliche Verlauf des Gurtes (6b) beschrieben: Das eine Ende des Gurtes (6b) ist etwa im Scheitelpunkt der Brücke (2) befestigt, verläuft dann in vertikaler Richtung auf dem Patiententisch (1) zu, wird dann etwa in die Horizontale umgelenkt und liegt von der Unterseite an der Beinschale (5b) an, um dann auf der gegenüberliegenden Seite im Wesentlichen wieder vertikal nach oben auf die Brücke (2) zu geführt zu werden. Dort wird der Gurt (5b) in die Ebene der Brücke (2) umgelenkt und an der patientenfernen Stirnfläche entlang geführt und dort manuell, im gezeigten Beispiel über einen Klettverschluss festgelegt.

In der gleichen, durch den Gurt (6b) definierten Ebene befindet sich ein weiterer Gurt (6a) mit einem zur Mittelebene der gesamten Anordnung und zum Gurt (6a) spiegelsymmetrischen Verlaufs. Diesem Gurt (6a) ist die Beinschale (5a) und der entsprechende Unterschenkel (4a) zugeordnet.

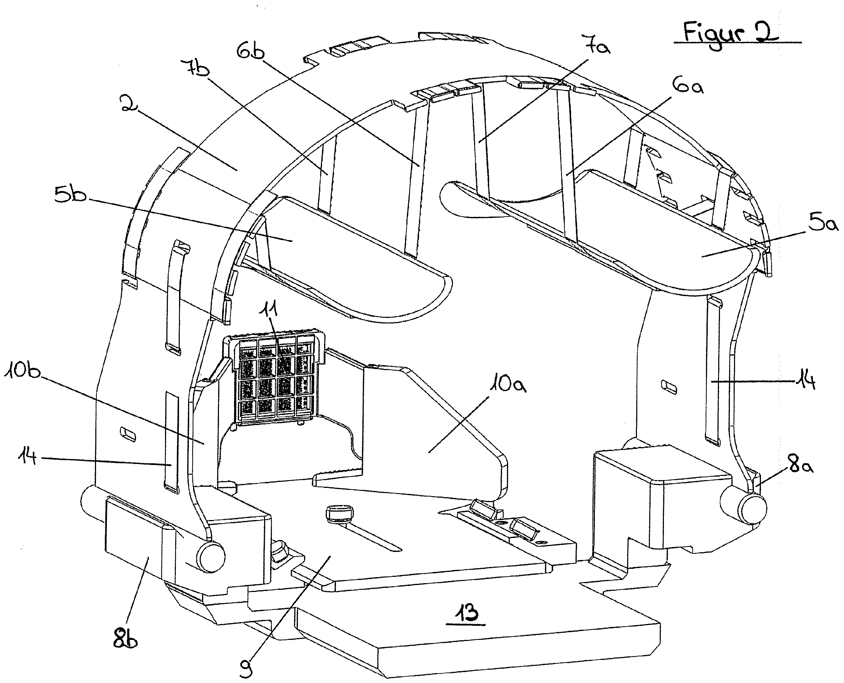

Auf der dem Probanden (3) zugewandten Stirnseite der Brücke (2) finden sich ebenfalls und zusätzlich Gurte (7), die in dieser zeichnerischer Darstellung nicht zu erkennen sind und erst in Figur 2 dargestellt und beschrieben sind.

An der Brücke im Übergangsbereich zum Patiententisch (1) hin findet sich jeweils eine Spannvorrichtung (8), die dazu dient, einen Gurte (14) in axialer Richtung zu spannen, dessen gegenüberliegendes Ende im wesentlichen horizontal an die Beinschale (5) herangeführt und dort befestigt ist. Bei axialem Zug vermittelt der Gurt (14) eine translatorische Bewegung nach außen und nähert den Unterschenkel (4) an die Brücke (2) an, so dass sich der Zugang zum Patienten aufgrund der weiteren Spreizung der Beine verbessert.

An einem relativ zum Patiententisch (1) verschiebbaren Schlitten (9) befinden sich noch zwei auf den Probanden (3) spitz zulaufende und in vertikaler Anordnung zum Patiententisch (1) verlaufende Stützen (10), an deren Scheitelpunkt sich ein Gitter (11) befindet, das in dem gezeigten, herangefahrenen Zustand einen transperinealen Zugang mit gezielter Ausrichtung möglich macht, wie es zur Entnahme von Gewebeproben aus dem Unterlaib und insbesondere der Prostata erforderlich ist. Die Einzelheiten des Schlittens (9) mit den Stützen (10) sowie dem Gitter (11) lassen sich der nachfolgenden Figur 2 deutlicher entnehmen.

Der Proband (3) liegt mit seinem Rücken auf dem Patiententisch (1) auf und die Arme sind längsseits an den Körper gelegt. Die Oberschenkel (12) bilden mit der durch den Patiententisch (1) definierten horizontalen Ebene einen spitzen Winkel. Die Unterschenkel (4) sind jeweils in einer Beinschale (5) abgestützt, die in einer horizontalen Ebene parallel zum Patiententisch (1) verläuft. Die Aufhängung und Abstützung der Beinschalen (5) erfolgt über die Brücke (2). Aus der Darstellung in Figur 1 ist gut zu erkennen, dass die Außenkonturen des Probanden (3) vollständig innerhalb einer durch die Brücke (2) beschriebenen Einhüllenden liegen, mit der einzigen Ausnahme, dass die Füße etwa radial nach außen über die Einhüllende überstehen. Dies ist unproblematisch, weil der Proband (3) nur soweit in die hier nicht gezeigte Röhre eingefahren wird, dass die Füße in Fahrtrichtung des Patiententisches nach außen zu überstehen, da sie nicht Gegenstand der Abbildung und Untersuchung sind. In den Patiententisch (1) eingelassen ist eine Bodenplatte mit dem darauf befindlichen Schlitten (9).

Wie bereits angekündigt, sind in **Figur 2** in perspektivische Darstellung die erfindungsgemäße Brücke (2) mit dem Schlitten (9) deutlieher zu erkennen, da die Darstellung nicht mit dem Probanden (3) und dem Patiententisch (1) überfrachtet ist.

Die Brücke (2) ist in etwa von C-förmiger Gestalt und endet jeweils an einer Spannvorrichtung (8a, 8b), die randseitig auf dem nicht dargestellten Patiententisch (1) ruhen und welche die Gurte (14) zur horizontalen Verschiebung der Beinschalen (5) in der Lage ist. Die Aufhängung der Beinschalen (5) mithilfe der Gurte (6 und 7) ist nunmehr klar und deutlich zu erkennen.

Die Aufhängung der Beinschale (5a) geschieht wie folgt:
In der später patientenfernen Stirnfläche der Brücke (2) verläuft der Gurt (6a), der oben im Bereich des Scheitels der Brücke (2) befestigt ist, dann nahezu vertikal nach unten verläuft, auf der Unterseite der Beinschale (5a) entlang und anschließend dazu vertikal nach oben geführt ist und mit Erreichen der Brücke (2) tangential nach unten zu umgelenkt wird und das Ende durch Umschlagen auf sich selbst über einen Klettverschluss festgelegt wird. In der später Probandennahen Stirnfläche und einem Abstand etwa in der axialen Breite der Brücke (2) verläuft ein weiterer Gurt (7a), der im Wesentlichen parallel zum Gurt (6a) jedoch im axialen Abstand hierzu angeordnet ist. Beide Gurte (6, 7) sind derart eingestellt, dass die Beinschale (5a, 6a) in der Horizontalen ausgerichtet ist. Die Spannvorrichtung (8a) dient der Anspannung der Gurte (14) und der horizontalen Ausrichtung der Beinschalen (5). Spiegelbildlich zur Längsachse der Brücke (2) findet sich zur eben beschriebenen Anordnung eine weitere identische Anordnung, so dass man im Ergebnis zwei parallel zueinander ausgerichtete Beinschalen (5a, 5b) findet, die der Aufnahme der beiden Unterschenkel des Probanden (3) dienen.

Die beiden Enden der Brücke (2) stehen über einer Bodenplatte (13) miteinander in Verbindung, mit deren Hilfe die gesamte Vorrichtung in den hier nicht gezeigten Patiententisch (1) eingelassen wird. Auf der Bodenplatte (13) ist der Schlitten (9) in einer senkrecht zu der durch die Brücke (2) beschriebenen Ebene verschiebbar. Der Schlitten (9) ist an seinem patientenseitigen Ende mit zwei Stützen (10a, 10b) in der Weise verbunden, dass die beiden Stützen in V-Form d. h. im spitzen Winkel zueinander verlaufen und senkrecht zu der durch den Schlitten (9) beschriebenen Ebene ausgerichtet sind. Die Aufgabe der Stützen (11) besteht darin, die Oberschenkel (12) des Patienten radial auseinander zu pressen, um die Zugänglichkeit für den operativen Eingriff zu verbessern. Der Übergangsbereich zwischen den Schenkeln bildet das Gitter (11), mit dessen Hilfe die Orientierung und Lokalisierung der Einführung des chirurgischen Instrumentes also insbesondere einer Biopsienadel in transperinealer Weise erfolgt. Der Eingriff wird unter Kontrolle und Überwachung durch bildgebende Verfahren durchgeführt. Die Art und Weise der Durchführung der chirurgischen Eingriffe ist an sich bekannt. Gegenstand vorliegender Anmeldung ist jedoch die Schaffung einer Vorrichtung, die es erlaubt, den Probanden in einer Position zu verbringen, welche die Herstellung von Bildern mit optimaler Abbildungsqualität gerade jener Bereiche zur Verfügung stellt, die für den Arzt von primärem Interesse sind.

Abschließend ist darauf hinzuweisen, dass die in den Figuren gezeigte Anwendung der erfindungsgemäßen Brücke (2) auf Anhebung der Unterschenkel (4) einen Spezialfall betrifft und in der Praxis hierauf nicht eingeschränkt wurde, so dass auch das Anheben und Ausrichten anderer Körperpartien auf analoger Weise durch Verwendung der Brücke (2) vorgenommen werden kann.

### Bezugszeichenliste

- 1: Patiententisch
- 2: Brücke
- 3: Proband
- 4a, 4b: Unterschenkel
- 5a, 5b: Beinschale
- 6a, 6b: (Probandenferne) Gurte
- 7a, 7b: (Probandennahe) Gurte
- 8: Spannvorrichtung
- 9: Schlitten
- 10,10b: Stützen
- 11: Gitter
- 12a,12b: Oberschenkel
- 13: Bodenplatte
- 14: Gurt

## Patentansprüche

1. Patiententisch für eine optimale Ausrichtung der abzubildenden Körperpartien eines Probanden in einer NMR-Röhre oder CT-Röhre, wobei der Patiententisch (1) in axialer Richtung in die Röhre einfahrbar ist, wobei
- eine Brücke (2) vorhanden ist,
- die Außenabmessungen der Brücke (2) in radialer Richtung kleiner gewählt sind als der lichte Durchmesser der Röhre,
- ein Gurt vorhanden ist, der mit seinem einen Ende an der Brücke (2) befestigt und in axialer Richtung spann- und fixierbar ist,
**dadurch gekennzeichnet, dass**
- die Brücke sich auf den Längsrändern des Patiententisches (1) in einander gegenüberliegenden Punkten abstützt, und
- der Gurt zur Anhebung und Festlegung einer zu untersuchenden und abzubildenden Körperregion unter der abzubildenden Körperregion hindurch und wieder zurück zur Brücke (2) geführt ist.

2. Vorrichtung nach Anspruch 1, **dadurch gekennzeichnet, dass** zwei Gurte (6a, 6b) vorhanden sind, die in der Ebene der Brücke (2), die senkrecht zur Längsachse des Patiententisches (1) verläuft, hintereinander liegend angeordnet sind.

3. Vorrichtung nach Anspruch 1 oder 2, **dadurch gekennzeichnet, dass** in derselben Brücke (2) zwei Gurte (6, 7) vorhanden sind, die parallel und in axialer Richtung des Patiententisches (1) versetzt zueinander angeordnet sind.

4. Vorrichtung nach einem der vorhergehenden Ansprüche, **dadurch gekennzeichnet, dass** die Brücke (2) auf dem Patiententisch (1) verfahrbar ist.

5. Vorrichtung nach einem der vorhergehenden Ansprüche, **dadurch gekennzeichnet, dass** mehrere Brücken (2) auf dem Patiententisch (1) angeordnet sind.

6. Vorrichtung nach einem der vorhergehenden Ansprüche, **dadurch gekennzeichnet, dass** das Material der Brücke (2) und/oder des Gurtes (6, 7) aus für Röntgenstrahlen und/oder Radiowellen transparentem Material, insbesondere GFK, besteht.

7. Vorrichtung nach einem der vorhergehenden Ansprüche, **dadurch gekennzeichnet, dass** zwischen Gurt (6, 7) und Körperpartie eine in seiner Form an die jeweils zu unterstützende Körperpartie angepasster Schale (5) angebracht ist.

8. Vorrichtung nach Anspruch 3 oder 5, **dadurch gekennzeichnet, dass** das Anspannen der Gurte (6, 7) in der Weise erfolgt, dass die unterstützende Körperpartie, wie zum Beispiel das Bein, nach außen angewinkelt und in Form eines "V" ausgerichtet ist.

9. Vorrichtung nach einem der vorhergehenden Ansprüche, **dadurch gekennzeichnet, dass** in der Ebene der Brücke (2) in axialer Richtung verlaufende Bolzen angeordnet sind, an denen das Ende eines Gurtes (6, 7) befestigbar und/oder der Gurt (6, 7) umlenkbar ist.

10. Verfahren zur Verwendung der Vorrichtung nach einem der vorhergehenden Ansprüche, **dadurch gekennzeichnet, dass**
- als erstes die Brücke (2) und der auf der Patientenliege befindlichen Proband (3), ggf. nach Verfahren der Brücke, einander zugeordnet und die anzuhebende Körperpartie im Bereich der Brücke (2) ausgerichtet wird,
- dass der Gurt (6, 7) unter der Körperpartie durchgefädelt und mit beiden Enden nach oben zu geführt wird und dessen eines Ende an der Brücke (2) befestigt und dessen anderes Ende durch axiales Spannen die Körperpartie soweit anhebt, dass die abzubildende Körperpartie jene räumlichen Position annimmt, die zur Herstellung einer optimalen Abbildung erforderlich ist,
- anschließend die Patientenliege in die Röhre des Aufnahme gerätes eingefahren und
- dann in üblicher Weise die Aufnahmen erstellt werden.

11. Verfahren nach Anspruch 10, **dadurch gekennzeichnet, dass** das Durchfädeln des Gurtes (6, 7) dadurch geschieht, dass ein Ende des Gurtes (6, 7) zunächst frei ist und anschließend nach dem Durchfädeln an der Brücke (2) lösbar befestigt wird und das axiale Spannen des Gurtes (6, 7) manuell oder mit einer Spannvorrichtung vorgenommen wird und das zu vorgenanntem gegenüberliegende Ende des Gurtes (6, 7) anschließend lösbar festgelegt wird.

12. Verfahren nach Anspruch 10, **dadurch gekennzeichnet, dass** beide Enden an der Brücke (2) befestigt sind und die hierdurch gebildete lose Schlaufe unterhalb der Körperpartie dadurch angebracht wird, dass der Proband (3) angehoben und dann durch Verfahren der Brücke (2) die Schlaufe unter die Körperpartie verbracht und durch Spannen der Gurte (6, 7) bis in die zur Abbildung optimale/gewünschte Position angehoben wird.

## Claims

1. Patient table for optimum orientation of those body parts of a test subject to be imaged in an NMR tube or CT tube, wherein the patient table (1) can be traversed into the tube in the axial direction,
- a bridge (2) being present,
- the outer dimensions of the bridge (2) being chosen such that they are smaller in the radial direction than the clear diameter of the tube,
- a strap being provided, which is fastened with one end on the bridge (2) and which can be tensioned and fixed in the axial direction,
**characterised in that**
- the bridge is supported on the longitudinal edges of the patient table (1) at points opposite one another, and,
- for lifting and fixing a body region to be examined and imaged, the strap is passed below the body region to be imaged and back to the bridge (2) again.

2. Device according to claim 1, **characterised in that** two straps (6a, 6b) are present, which are disposed lying one behind the other in the plane of the bridge (2), which runs perpendicular to the longitudinal axis of the patient table (1),

3. Device according to claim 1 or 2, **characterised in that,** two straps (6, 7) are present in the same bridge (2), and are disposed such that they are parallel to one another and offset with respect to one another in the axial direction of the patient table (1).

4. Device according to any one of the preceding claims, **characterised in that** the bridge (2) can be traversed on the patient table (1).

5. Device according to any one of the preceding claims, **characterised in that** a plurality of bridges (2) are disposed on the patient table (1).

6. Device according to any one of the preceding claims, **characterised in that** the material of the bridge (2) and/or of the strap (6, 7) consists of a material that is transparent to X-rays and/or radio waves, in particular GRP.

7. Device according to any one of the preceding claims, **characterised in that,** between the strap (6, 7) and body part, a tray (5) is mounted, of which the form is adapted to the body part in each case that is to be supported.

8. Device according to claim 3 or 5, **characterised in that** the tensioning of the straps (6, 7) takes place in such a manner that the body part to be supported, such as, for example, the leg, is angled outwardly and is oriented in the form of a "V".

9. Device according to any one of the preceding claims, **characterised in that,** in the plane of the bridge (2) bolts, which extend in the axial direction, are disposed, to which the end of a strap (6, 7) can be fastened, and/or at which the strap (6, 7) can be deflected.

10. Method for using the device according to any one of the preceding claims, **characterised in that**
- first, the bridge (2) and the test subject (3), who is located on the patient table, are assigned to one another after traversing of the bridge if appropriate, and the body part to be lifted is oriented in the region of the bridge (2),
- the strap (6, 7) is threaded below the body part and is guided upwardly at both ends, and one of its ends is fastened on the bridge (2) and the other of its ends, by axial tensioning of the strap, lifts the body part to such an extent that the body part to be imaged assumes that spatial position that is necessary to produce an optimum image,
- subsequently the patient table is traversed into the tube of the recording device and
- the recordings are then produced in the conventional manner.

11. Method according to Claim 10, **characterised in that** the threading of the strap (6, 7) is performed **in that** one end of the strap (6, 7) is at first free and then, after threading through, is detachably fastened on the bridge (2) and the axial tensioning of the strap (6, 7) is performed manually or with a tensioning device and the said opposite end of the strap (6, 7) is subsequently detachably fixed.

12. Method according to Claim 10, **characterised in that** both ends are fastened on the bridge (2) and the loose loop that is thereby formed is attached below the body part such that the test subject (3) is lifted and then, by traversing of the bridge (2), the loop is passed below the body part and, by tensioning of the straps (6, 7), is raised into the optimum/desired position for the imaging.

## Revendications

1. Table médicale destinée à orienter de façon optimale les parties du corps devant être imagées d'une personne examinée dans un tube d'appareil à RMN ou un tube d'appareil de tomographie par ordinateur, sachant que la table médicale (1) peut être rentrée dans le tube dans le sens axial, sachant
- qu'il existe un pont (2),
- que les dimensions extérieures de ce pont (2) ont été choisies, dans le sens radial, plus petites que le diamètre intérieur du tube,
- qu'il existe une sangle qui est, à l'une de ses extrémités, fixée au pont (2) et peut être tendue et fixée dans le sens axial,
**caractérisée par le fait**
- **que** le pont s'appuie sur des points situés les uns en face des autres sur les bords longitudinaux de la table médicale (1), et
- **que** la sangle servant à soulever et à fixer la région du corps devant être imagée est menée en dessous de la région du corps devant être imagée avant de retourner au pont (2).

2. Dispositif selon la revendication 1, **caractérisé par le fait qu'**il existe deux sangles (6a, 6b), qui sont disposées à plat l'une derrière l'autre dans le plan du pont (2), qui suit un parcours vertical par rapport à l'axe longitudinal de la table médicale (1).

3. Dispositif selon la revendication 1 ou 2, **caractérisé par le fait qu'**il y a deux sangles (6, 7) dans le même pont (2), lesquelles sont disposées parallèlement et dans le sens axial de la table médicale (1), décalées l'une par rapport à l'autre.

4. Dispositif selon une des revendications précédentes, **caractérisé par le fait que** le pont (2) peut être déplacé sur la table médicale (1).

5. Dispositif selon une des revendications précédentes, **caractérisé par le fait que** plusieurs ponts (2) sont disposés sur la table médicale (1).

6. Dispositif selon une des revendications précédentes, **caractérisé par le fait que** le matériau du pont (2) et/ou des sangles (6, 7) consiste en un matériau transparent par rapport aux rayons X et/ou les ondes radio, notamment du plastique à renfort de verre.

7. Dispositif selon une des revendications précédentes, **caractérisé par le fait qu'**entre les sangles (6, 7) et la partie du corps est disposée une coque (5) dont la forme est adaptée à la partie du corps devant être soutenue dans chaque cas.

8. Dispositif selon la revendication 3 ou 5, **caractérisé par le fait que** le serrage des sangles (6, 7) est réalisé de manière à ce que la partie du corps devant être soutenue, comme par exemple la jambe, est orientée pliée vers l'extérieur en adoptant la forme d'un « V ».

9. Dispositif selon une des revendications précédentes, **caractérisé par le fait que** dans le plan du pont (2) sont disposés des goujons orientés dans le sens axial, auxquels l'extrémité d'une sangle (6, 7) peut être fixée et/ou la sangle (6, 7) peut être renvoyée.

10. Procédé destiné à utiliser le dispositif selon une des revendications précédentes, **caractérisé par le fait**
- **qu'**en premier lieu le pont (2) et la personne examinée (3) se trouvant sur la table médicale sont attribués l'un à l'autre, le cas échéant d'après le procédé du pont, et que la partie du corps devant être soulevée est orientée dans la zone du pont (2),
- **que** la sangle (6, 7) est enfilée en dessous de la partie du corps et est menée avec ses deux extrémités vers le haut, une des deux extrémités étant fixée sur le pont (2) et l'autre extrémité soulevant la partie du corps par serrage axial jusqu'à ce que la partie du corps à imager adopte la position spatiale qui est nécessaire pour réaliser une image optimale,
- la table médicale étant ensuite rentrée dans le tube de l'appareil d'enregistrement,
- les prises étant ensuite réalisées de la manière habituelle.

11. Procédé selon la revendication 10, **caractérisé par le fait que** l'enfilage des sangles (6, 7) est réalisé en ce qu'une extrémité des sangles (6, 7) est d'abord libre avant d'être ensuite fixée au pont (2) de façon à pouvoir être détachée, le serrage axial de la sangle (6, 7) étant ensuite réalisé manuellement ou avec un dispositif de serrage, l'extrémité opposée des sangles (6, 7) précédemment nommée étant ensuite fixée de façon à pouvoir être détachée.

12. Procédé selon la revendication 10, **caractérisé par le fait que** les deux extrémités sont fixées sur le pont (2) et que la boucle lâche formée de cette manière est placée en dessous de la partie du corps en ce que la personne à examiner (3) est soulevée et que la boucle est ensuite placée sous la partie du corps en recourant au procédé du pont (2), cette dernière étant soulevée dans la position optimale/souhaitée pour l'imagerie en serrant les sangles (6, 7).
